# DEMANDE DE BREVET EUROPEEN

(11) **EP 1 374 809 A1**
(43) Date de publication de la demande: **02.01.2004**
(21) Numéro de dépôt: 03291438.4
(22) Date de dépôt: 16.06.2003
(51) Int. Cl.: A61F 2/44

(54) **Cage intervertébrale perfectionnée**

(30) Priorité: 25.06.2002 FR 0207815
(71) Demandeur: Razian, Hassan, 94240 l'Hay les Roses (FR)
(72) Inventeur: Razian, Hassan, 94240 l'Hay les Roses (FR)
(74) Mandataire: Flavenot, Bernard

(57) **Abrégé**

La présente invention concerne les cages intervertébrales, notamment pour le traitement du rachis dégénératif.

La cage intervertébrale selon l'invention se caractérise essentiellement par le fait qu'elle comporte une entretoise 1 apte à être disposée entre deux vertèbres, une lame 20 comportant deux extrémités opposées 25, 26, des moyens 21 pour déplacer la lame par rapport à l'entretoise de façon que la lame soit apte à prendre deux positions, une première position dans laquelle la lame est située dans l'espace 22 compris entre les deux plans 23, 24 contenant les deux faces 3, 4 de l'entretoise, et une seconde position dans laquelle les deux extrémités opposées 25, 26 de la lame émergent de part et d'autre de cet espace 22, les deux extrémités 25, 26 comportant chacune un épaulement, de façon que ces épaulements 41, 42 soient enfoncés dans les deux corps vertébraux quand ladite lame 20 est dans sa seconde position.

## Description

La présente invention concerne des perfectionnements aux cages intervertébrales qui trouvent une application particulièrement avantageuse, mais non exclusivement, pour le traitement du rachis dégénératif.

Une cage intervertébrale, notamment pour le traitement du rachis dégénératif, est déjà connue. Une telle cage est par exemple décrite dans le document EP 1 104 665. Elle comporte essentiellement une entretoise en forme de disque comprenant deux faces de base opposées sensiblement planes et parallèles et une paroi latérale reliant les deux faces de base. Cette entretoise est apte à être disposée entre les faces en regard des deux corps vertébraux respectivement de deux vertèbres consécutives, en remplacement du disque endommagé situé entre ces deux vertèbres, les deux faces de base de l'entretoise étant placées au contact des corps vertébraux. L'entretoise peut en outre comporter une cavité ouverte dans laquelle il est possible de placer un greffon osseux ou analogue dans le but de souder entre eux les deux corps vertébraux par ostéosynthèse. La cage comprend aussi au moins une lame, comportant deux extrémités opposées conformées en biseau, et des moyens pour déplacer cette lame par rapport à une première des deux parties de paroi latérale de façon que la lame soit apte à prendre deux positions, une première position dans laquelle la lame est entièrement située dans l'espace compris entre les deux premier et second plans contenant les deux faces de base de l'entretoise, et une seconde position dans laquelle les deux extrémités opposées de la lame émergent de part et d'autre de cet espace.

Cette réalisation donne généralement de bons résultats, pour notamment maintenir les deux corps vertébraux à bonne distance l'un de l'autre et empêcher, du fait notamment de la présence de la lame qui s'implante dans les deux corps vertébraux, que la cage subisse des glissements suivant une direction sensiblement perpendiculaire à l'axe général de la colonne vertébrale. En revanche, elle n'empêche pas que les deux corps vertébraux puissent s'écarter l'un de l'autre. Pour pallier cet inconvénient, avec une telle cage, il est implanté en plus par exemple des plaques, tiges, etc. qui sont vissées dans les deux corps vertébraux.

La présente invention a pour but de réaliser un perfectionnement à la cage intervertébrale pour le traitement du rachis dégénératif telle que définie ci-avant, qui permet de ne pas implanter d'autres éléments comme des plaques, tiges, etc. comme mentionné ci-dessus.

Plus précisément, la présente invention a pour objet une cage intervertébrale pour le traitement du rachis dégénératif, comprenant:
une entretoise sensiblement en forme de disque comportant deux faces de base opposées sensiblement planes et parallèles et une paroi latérale reliant les deux faces de base, cette paroi latérale comportant deux parties opposées, ladite entretoise étant apte à être disposée entre les faces en regard des deux corps vertébraux respectivement de deux vertèbres consécutives en remplacement du disque endommagé entre ces deux vertèbres, les deux faces de base de l'entretoise étant aptes à être placées au contact au moins partiel des deux corps vertébraux,
   - au moins une lame, ladite lame comportant deux extrémités opposées conformées en biseau, et
   - des moyens pour déplacer ladite lame par rapport à une première des deux parties de paroi latérale de façon que la lame soit apte à prendre deux positions :
      * une première position dans laquelle la lame est entièrement située dans l'espace compris entre les deux premier et second plans contenant les deux faces de base de l'entretoise, et
      * une seconde position dans laquelle les deux extrémités opposées de ladite lame émergent de part et d'autre dudit espace,
caractérisée par le fait que les deux extrémités opposées de ladite lame émergeant de part et d'autre dudit espace lorsque la lame est dans sa seconde position, comportent chacune un épaulement, ces deux dits épaulement étant respectivement réalisés sur les deux extrémités opposées de façon qu'ils soient sensiblement contenus dans des plans parallèles aux plans des deux faces de base opposées de l'entretoise et respectivement enfoncés dans les deux corps vertébraux quand ladite lame est dans sa seconde position.

D'autres caractéristiques et avantages de l'invention apparaîtront au cours de la description suivante donnée en regard des dessins annexés à titre illustratif mais nullement limitatif, dans lesquels :
La figure 1 représente une vue partielle d'un premier mode de réalisation de la cage intervertébrale selon l'invention, et
Les figures 2A, 2B et 2C représentent trois vues, respectivement de côté, de face et de dessus, d'une partie d'un second mode de réalisation de la cage intervertébrale selon l'invention.

Il est bien précisé que, sur les figures, les mêmes références désignent les mêmes éléments, quelle que soit la figure sur laquelle elles apparaissent et quelle que soit la forme de représentation de ces éléments.

De même, si des éléments ne sont pas spécifiquement référencés sur l'une des figures, leurs références peuvent être aisément retrouvées en se reportant à une autre figure.

Le Demandeur tient aussi à préciser que les figures représentent deux modes de réalisation de l'objet selon l'invention, mais qu'il peut exister d'autres modes de réalisation qui répondent à la définition de cette invention.

Il précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si les modes de réalisation de l'objet selon l'invention tel qu'illustrés comportent plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

La présente invention concerne une cage intervertébrale perfectionnée pour, notamment, le traitement du rachis dégénératif.

Cette cage intervertébrale perfectionnée comprend essentiellement une entretoise 1 en forme de disque sensiblement parallélépipédique comportant avantageusement un logement en creux dans lequel il est possible de placer un greffon osseux, comme notamment décrit dans le document définissant l'art antérieur cité dans le préambule de la présente description.

De ce fait, ce disque comporte deux faces de base opposées 3, 4 sensiblement planes et parallèles entre elles et une paroi latérale 5 reliant les deux faces de base. Dans une forme particulièrement avantageuse, cette paroi latérale comporte par exemple deux parties opposées dont, seule, la partie référencée 6 est représentée.

L'entretoise 1 est apte à être disposée entre les faces en regard 8, 9 des deux corps vertébraux 10, 11 respectivement de deux vertèbres consécutives, en remplacement du disque endommagé qui se trouvait entre ces deux vertèbres, les deux faces de base 3, 4 de l'entretoise 1 étant aptes à être placées au contact au moins partiel des faces en regard 8, 9 des deux corps vertébraux.

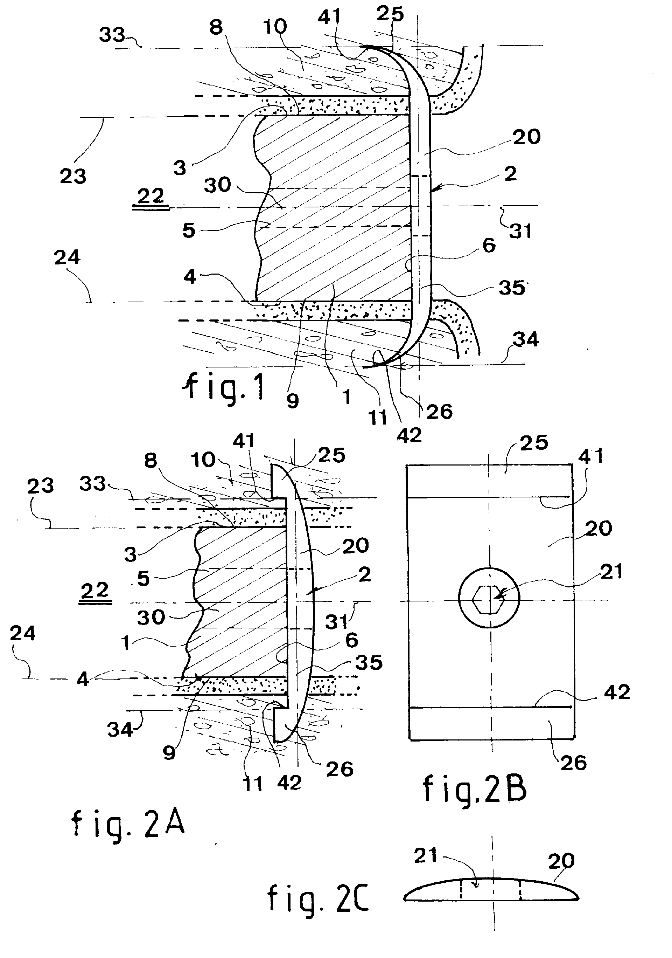

La cage comporte en outre au moins une lame 20 et des moyens 21 pour déplacer cette lame par rapport à la première des deux parties opposées de la paroi latérale 5, en l'occurrence la partie 6 sur la figure 1, de façon que la lame puisse prendre deux positions:
- une première position dans laquelle la lame est entièrement située dans l'espace 22 compris entre les deux premier et second plans 23, 24 contenant les deux faces de base 3, 4 de l'entretoise 1, et
- une seconde position comme celle qui est illustrée sur les figures 1 et 2A, dans laquelle les deux extrémités opposées 25, 26 de la lame émergent de part et d'autres de cet espace 22.

En outre, de façon avantageuse, les deux extrémités 25, 26 de la lame 20 sont conformées en biseau de façon à pouvoir pénétrer plus facilement dans l'os des corps vertébraux 10, 11 à la manière d'une lame de couteau.

Dans une réalisation avantageuse, les moyens 21 définis ci-dessus, pour déplacer la lame 20 par rapport à une première des deux parties de paroi latérale opposées, en l'occurrence la partie 6 dans la représentation sur la figure 1, de façon que la lame soit apte à apprendre deux positions, comportent par exemple un arbre 30 monté au moins en rotation suivant un axe 31 dans la première partie 6 de la paroi latérale 5, et des moyens pour monter la lame en coopération avec une première extrémité de l'arbre 30, la seconde extrémité de l'arbre plongeant par exemple dans le logement creux défini ci-avant.

Cet arbre 30 permet de transmettre la commande pour faire passer la lame 20 de sa première position à sa seconde position, et réciproquement s'il est par exemple nécessaire de retirer la cage intervertébrale après qu'elle ait été implantée.

Les moyens pour faire passer, par rotation, la lame 20 de sa première position à sa seconde position et réciproquement, peuvent être constitués par des parties en saillie ou en creux de forme polygonale réalisées à la première extrémité de l'arbre 30, avec lesquelles peuvent coopérer des ancillaires sensiblement identiques à des outils classiques que l'on dénomme couramment clés à tube, tourne vis ou analogues.

La cage peut aussi comporter des moyens pour maintenir la lame dans sa seconde position et l'empêcher de revenir à sa première position sous l'effet de quelque contrainte que ce soit qui serait exercée par le patient lui-même, tout en lui permettant de revenir à cette première position sous l'effet de forces extérieures exercées, par exemple, à l'aide d'un instrument ancillaire.

Selon une caractéristique importante de l'invention, les deux extrémités opposées 25, 26 de la lame émergeant de part et d'autre de l'espace 22 lorsque la lame 20 est dans sa seconde position, comportent chacune un épaulement 41, 42, ces deux épaulements étant respectivement réalisés sur les deux extrémités opposées 25, 26 de façon que, lorsque la lame 20 est dans sa seconde position, figures 1 et 2A, ils soient sensiblement contenus, dans des plans 33, 34 parallèles aux plans 23, 24 des deux faces de base opposées 3, 4 de l'entretoise et respectivement enfoncés dans les deux corps vertébraux 10, 11.

De cette façon, quand la lame 20 est dans sa seconde position, ses deux extrémités 25, 26 avec leur épaulement respectif 41, 42 implantées dans l'os des corps vertébraux 10, 11 assurent simultanément deux fonctions, à savoir : empêcher le glissement de la cage suivant l'axe 31 et s'opposer à l'écartement des deux corps vertébraux 10, 11 perpendiculairement à ce même axe 31. La lame 20 joue donc une fonction d'agrafe que les cages de l'art antérieur n'avaient pas.

La lame peut être sensiblement plate, comme illustré sur les figures, ou courbée ou équivalent, mais présentant dans son ensemble une forme générale définie un plan, comme le plan 35, sensiblement perpendiculaire à l'axe 31.

Dans les deux modes de réalisation illustrés, les deux épaulements sont disposés d'un même côté du plan général 35 de la lame 20, la section transversale de la lame dans son ensemble se présentant alors sous la forme d'un **U** ou équivalent.

Il est cependant précisé que les deux épaulements peuvent être disposés de part et d'autre du plan général 35 de la lame 20, la section transversale de la lame 20 dans son ensemble se présentant alors sous la forme d'un **Z** ou équivalent.

Il est aussi possible que la lame comporte quatre épaulements, deux respectivement de part et d'autre du plan général 35 de la lame 20, la section transversale de la lame 20 dans son ensemble se présentant alors sous la forme d'un **I** ou équivalent.

Ces épaulements 41, 42 peuvent être réalisés de différentes façons. Les deux modes de réalisation représentés sur les figures illustrent deux de ces façons.

La figure 1 représente un mode de réalisation de ces deux épaulements dans le cas d'une lame en forme de **U**, dans lequel la lame est formée d'une seule pièce obtenue par pliage ou moulage ou formage de façon que les deux extrémités 25, 26 soit courbées et en déport d'un même côté par rapport au plan 35 de la lame.

Les figures 2A à 2C représentent un autre un mode de réalisation de ces deux épaulements, toujours dans le cas d'une lame en forme de **U**. Dans ce cas, la lame est obtenue à partir, par exemple, d'un secteur de cylindre ou d'un segment de sphère, dans la base duquel est usinée une rainure à section transversale définie dans un plan contenant l'axe 31 et avantageusement sensiblement rectangulaire, de façon que les deux bords opposés de cette rainure forment les deux épaulements 41, 42.

L'ensemble des figures représente la lame 20 dans sa seconde position comme décrit auparavant.

Cependant, quand elle est dans sa première position, les épaulements 41, 42 peuvent se placer de part et d'autre de l'entretoise 1 et venir, par exemple, se plaquer contre sa paroi latérale 5 qui présentera alors avantageusement, à ce niveau, une forme complémentaire de celle des faces courbes des extrémités 25, 26 de la lame, ces extrémités pouvant aussi se positionner dans des logements en creux réalisés dans cette paroi latérale 5.

Il est en outre précisé que la cage selon l'invention peut comporter d'autres caractéristiques en plus de celles définies ci-dessus. Ces autres caractéristiques n'ont pas été décrites ici car elles n'entrent pas dans le champ de la présente invention.

## Revendications

1. Cage intervertébrale pour le traitement du rachis dégénératif, comprenant:
une entretoise (1) sensiblement en forme de disque comportant deux faces de base opposées (3, 4) sensiblement planes et parallèles et une paroi latérale (5) reliant les deux faces de base, cette paroi latérale comportant deux parties opposées (6, 7), ladite entretoise étant apte à être disposée entre les faces en regard (8, 9) des deux corps vertébraux (10, 11) respectivement de deux vertèbres consécutives en remplacement du disque endommagé entre ces deux vertèbres, les deux faces de base de l'entretoise étant aptes à être placées au contact au moins partiel des deux corps vertébraux,
- au moins une lame (20), ladite lame comportant deux extrémités opposées (25, 26) conformées en biseau, et
- des moyens (21) pour déplacer ladite lame par rapport à une première (6) des deux parties de paroi latérale de façon que la lame soit apte à prendre deux positions :
* une première position dans laquelle la lame est entièrement située dans l'espace (22) compris entre les deux premier et second plans (23, 24) contenant les deux faces de base (3, 4) de l'entretoise (1), et
* une seconde position dans laquelle les deux extrémités opposées (25, 26) de ladite lame émergent de part et d'autre dudit espace (22),
**caractérisée par le fait que** les deux extrémités opposées (25, 26) de ladite lame émergeant de part et d'autre dudit espace (22) lorsque la lame est dans sa seconde position, comportent chacune un épaulement, ces deux épaulements (41, 42) étant respectivement réalisés sur les deux extrémités opposées (25, 26) de façon qu'ils soient sensiblement contenus dans des plans parallèles aux plans des deux faces de base opposées (3, 4) de l'entretoise et respectivement enfoncés dans les deux corps vertébraux quand ladite lame (20) est dans sa seconde position.

2. Cage intervertébrale selon la revendication 1, **caractérisée par le fait que** ladite lame (20) dans son ensemble se présente sous la forme d'un **U**.

3. Cage intervertébrale selon la revendication 1, **caractérisée par le fait que** la lame (20) dans son ensemble se présente sous la forme d'un **Z**.

4. Cage intervertébrale selon la revendication 1, **caractérisée par le fait que** la lame (20) dans son ensemble se présente sous la forme d'un **I**.

5. Cage intervertébrale selon l'une des revendications 1 à 4, **caractérisée par le fait que** ladite lame (20) avec lesdits épaulements (41, 42) est réalisée d'une seule pièce par l'un des moyens suivants : pliage, moulage, formage, de façon que les deux extrémités (25, 26) de la lame soit courbées et en déport par rapport au plan (35) de ladite lame (20).

6. Cage intervertébrale selon la revendication 2, **caractérisée par le fait que** ladite lame (20) est obtenue à partir de l'un des éléments suivants : un secteur de cylindre, un segment de sphère, dans la base duquel est usinée une rainure à section transversale sensiblement rectangulaire, de façon que les deux bords opposés de cette dite rainure forment les deux dits épaulements (41, 42).

7. Cage intervertébrale selon l'une des revendications 1 à 6, **caractérisée par le fait que** les épaulements (41, 42), quand ladite lame (20) est dans sa première position, se placent de part et d'autre de l'entretoise (1) en venant se plaquer contre sa paroi latérale (5).

8. Cage intervertébrale selon l'une des revendications 1 à 7, **caractérisée par le fait que** lesdites extrémités opposées (25, 26), quand ladite lame (20) est dans sa première position, se positionnent dans des logements en creux réalisés dans ladite paroi latérale (5).
